# EUROPEAN PATENT APPLICATION

(11) **EP 4 223 305 A1**
(43) Date of publication of application: **09.08.2023**
(21) Application number: 21875522.1
(22) Date of filing: 27.09.2021
(51) Int. Cl.: A61K 38/16, A61K 38/10, A61P 11/00, A61P 17/00, A61P 43/00, C07K 14/525

(54) **WNT SIGNALING PATHWAY INHIBITOR**

(30) Priority: 02.10.2020 JP 2020168029
(71) Applicant: OSAKA UNIVERSITY, Suita-shi Osaka 565-0871 (JP)
(72) Inventor: SHIMAMURA, Munehisa, Suita-shi, Osaka 565-0871 (JP); NAKAGAMI, Hironori, Suita-shi, Osaka 565-0871 (JP); MORISHITA, Ryuichi, Suita-shi, Osaka 565-0871 (JP)
(74) Representative: Meissner Bolte Partnerschaft mbB
(86) International application number: PCT/JP2021/035368
(87) International publication number: WO 2022/071220

(57) **Abstract**

Provided are preventive or therapeutic agents for fibrotic diseases and cancer. A Wnt signaling pathway inhibitor or a preventive and therapeutic agent for fibrotic diseases or cancer, both comprising an oligopeptide consisting of an amino acid sequence containing a DE loop sequence of RANKL protein and a β-strand D sequence of RANKL protein placed adjacent to the N-terminal side of the DE loop sequence.

## Description

### Technical Field

The present invention relates to a Wnt signaling pathway inhibitor and the like.

### Background Art

Scleroderma is a disease in which skin sclerosis progresses and visceral lesions appear within 5 to 6 years after onset, and the number of patients with scleroderma is more than 20,000 in Japan alone. Steroids, cyclophosphamide, proton pump inhibitors, prostacyclin, ACE inhibitors, endothelin receptor antagonists, and the like have been used as treatments for scleroderma; however, they do not lead to fundamental treatment.

Idiopathic pulmonary fibrosis (IPF) has an average survival period of 3 to 5 years after confirmation of diagnosis and an average survival period of less than 2 months after acute exacerbation. Prevention and treatment are important. Pirfenidone, nintedanib, and the like are marketed as IPF therapeutic drugs; however, they do not lead to fundamental treatment.

The number of cancer patients is increasing with the progress of aging and changes in lifestyle habits. In particular, lung cancer has a poor prognosis, and the development of effective therapeutic agents has been desired.

PTL 1 has reported that RANKL peptide inhibits inflammatory cytokines via TLR. However, it is not known that RANKL peptide is effective for prevention or treatment of fibrotic diseases such as scleroderma and pulmonary fibrosis, and cancer.

### Citation List

### Patent Literature

PTL 1: WO2018/084311

### Non-Patent Literature

NPL 1: The Journal of Clinical Investigation, No. 7, Vol. 108, 2001, pages 971-979.
NPL 2: Mol Endocrinol, January 2009, 23(1): 35-46.

### Summary of Invention

### Technical Problem

An object of the present invention is to provide a preventive or therapeutic agent for fibrotic diseases and cancer.

### Solution to Problem

As a result of extensive research to achieve the above object, the present inventor found that an oligopeptide consisting of an amino acid sequence containing a DE loop sequence of RANKL protein and a β-strand D sequence of RANKL protein placed adjacent to the N-terminal side of the DE loop sequence can inhibit Wnt signaling pathway and suppress binding of LGR4 to RSPO1-3. The present inventor also found that based on this action, the oligopeptide has a preventive or therapeutic effect on fibrotic diseases and cancer. As a result of further research based on this finding, the present inventor has completed the present invention. Specifically, the present invention includes the following embodiments.

Item 1. A Wnt signaling pathway inhibitor comprising an oligopeptide consisting of an amino acid sequence containing a DE loop sequence of RANKL protein and a β-strand D sequence of RANKL protein placed adjacent to the N-terminal side of the DE loop sequence,
wherein the DE loop sequence is the following amino acid sequence (a) or (b) :
   (a) the amino acid sequence represented by SEQ ID NO: 1, or
   (b) an amino acid sequence with substitution, deletion, addition, or insertion of one amino acid in the amino acid sequence represented by SEQ ID NO: 1; and
the β-strand D sequence is the following amino acid sequence (c) or (d):
   (c) the amino acid sequence represented by any one of SEQ ID NOs: 2 to 5, or
   (d) an amino acid sequence with substitution, deletion, addition, or insertion of one to three amino acids in the amino acid sequence represented by any one of SEQ ID NOs: 2 to 5.

Item 2. The inhibitor according to Item 1, wherein the DE loop sequence is the amino acid sequence (a).

Item 3. The inhibitor according to Item 2, wherein the one to three amino acids are one or two amino acids.

Item 4. The inhibitor according to any one of Items 1 to 3, wherein in the amino acid sequence (d), the leucine residue at the N-terminus of SEQ ID NOs: 2 and 4, and the leucine residue that is the fourth amino acid residue from the N-terminus of SEQ ID NOs: 3 and 5, are not substituted or deleted.

Item 5. The inhibitor according to any one of Items 1 to 4, wherein the amino acid sequence of the oligopeptide contains a β-strand E sequence of RANKL protein placed adjacent to the C-terminal side of the DE loop sequence.

Item 6. The inhibitor according to Item 5, wherein the β-strand E sequence is the following amino acid sequence (e) or (f) :
(e) the amino acid sequence represented by any one of SEQ ID NOs: 6 to 9, or
(f) an amino acid sequence with substitution, deletion, addition, or insertion of one to three amino acids in the amino acid sequence represented by any one of SEQ ID NOs: 6 to 9.

Item 7. The inhibitor according to any one of Items 1 to 6, wherein the amino acid sequence of the oligopeptide is free from a CD loop sequence of RANKL protein.

Item 8. The inhibitor according to any one of Items 1 to 7, wherein the amino acid sequence of the oligopeptide is the following amino acid sequence (i) or (j):
(i) the amino acid sequence represented by any one of SEQ ID NOs: 12 to 21, or
(j) an amino acid sequence with substitution, deletion, addition, or insertion of one to three amino acids in the amino acid sequence represented by any one of SEQ ID NOs: 12 to 21.

Item 9. The inhibitor according to any one of Items 1 to 7, wherein the amino acid sequence of the oligopeptide is the following amino acid sequence (i') or (j'):
(i') the amino acid sequence represented by SEQ ID NO: 12, 17, 20, or 21, or
(j') an amino acid sequence with substitution, deletion, addition, or insertion of one to three amino acids in the amino acid sequence represented by SEQ ID NO: 12, 17, 20, or 21.

Item 10. The inhibitor according to any one of Items 1 to 9, wherein the oligopeptide has an acetylated N-terminus and an amidated C-terminus.

Item 11. The inhibitor according to any one of Items 1 to 10, wherein the oligopeptide has a length of 50 amino acid residues or less.

Item 12. The inhibitor according to Item 11, wherein the oligopeptide has a length of 40 amino acid residues or less.

Item 13. The inhibitor according to any one of Items 1 to 12, for use in prevention or treatment of a fibrotic disease, or for use in prevention or treatment of cancer.

Item 14. The inhibitor according to Item 13, wherein the fibrotic disease is a fibrotic disease in at least one member selected from the group consisting of skin and lung, and the cancer is lung cancer.

Item 15. A preventive or therapeutic agent for a fibrotic disease, the agent comprising an oligopeptide consisting of an amino acid sequence containing a DE loop sequence of RANKL protein and a β-strand D sequence of RANKL protein placed adjacent to the N-terminal side of the DE loop sequence,
wherein the DE loop sequence is the following amino acid sequence (a) or (b) :
   (a) the amino acid sequence represented by SEQ ID NO: 1, or
   (b) an amino acid sequence with substitution, deletion, addition, or insertion of one amino acid in the amino acid sequence represented by SEQ ID NO: 1; and
the β-strand D sequence is the following amino acid sequence (c) or (d) :
   (c) the amino acid sequence represented by any one of SEQ ID NOs: 2 to 5, or
   (d) an amino acid sequence with substitution, deletion, addition, or insertion of one to three amino acids in the amino acid sequence represented by any one of SEQ ID NOs: 2 to 5.

Item 16. A preventive or therapeutic agent for cancer, the agent comprising an oligopeptide consisting of an amino acid sequence containing a DE loop sequence of RANKL protein and a β-strand D sequence of RANKL protein placed adjacent to the N-terminal side of the DE loop sequence,
wherein the DE loop sequence is the following amino acid sequence (a) or (b) :
   (a) the amino acid sequence represented by SEQ ID NO: 1, or
   (b) an amino acid sequence with substitution, deletion, addition, or insertion of one amino acid in the amino acid sequence represented by SEQ ID NO: 1; and
the β-strand D sequence is the following amino acid sequence (c) or (d) :
   (c) the amino acid sequence represented by any one of SEQ ID NOs: 2 to 5, or
   (d) an amino acid sequence with substitution, deletion, addition, or insertion of one to three amino acids in the amino acid sequence represented by any one of SEQ ID NOs: 2 to 5.

Item 17. A method for inhibiting Wnt signaling pathway, the method comprising bringing an oligopeptide into contact with a cell, the oligopeptide consisting of an amino acid sequence containing a DE loop sequence of RANKL protein and a β-strand D sequence of RANKL protein placed adjacent to the N-terminal side of the DE loop sequence,
wherein the DE loop sequence is the following amino acid sequence (a) or (b) :
   (a) the amino acid sequence represented by SEQ ID NO: 1, or
   (b) an amino acid sequence with substitution, deletion, addition, or insertion of one amino acid in the amino acid sequence represented by SEQ ID NO: 1; and
the β-strand D sequence is the following amino acid sequence (c) or (d) :
   (c) the amino acid sequence represented by any one of SEQ ID NOs: 2 to 5, or
   (d) an amino acid sequence with substitution, deletion, addition, or insertion of one to three amino acids in the amino acid sequence represented by any one of SEQ ID NOs: 2 to 5.

Item 18. A method for preventing or treating a fibrotic disease or cancer, the method comprising administering an oligopeptide to a patient with a fibrotic disease or cancer, the oligopeptide consisting of an amino acid sequence containing a DE loop sequence of RANKL protein and a β-strand D sequence of RANKL protein placed adjacent to the N-terminal side of the DE loop sequence,
wherein the DE loop sequence is the following amino acid sequence (a) or (b) :
   (a) the amino acid sequence represented by SEQ ID NO: 1, or
   (b) an amino acid sequence with substitution, deletion, addition, or insertion of one amino acid in the amino acid sequence represented by SEQ ID NO: 1; and
the β-strand D sequence is the following amino acid sequence (c) or (d) :
   (c) the amino acid sequence represented by any one of SEQ ID NOs: 2 to 5, or
   (d) an amino acid sequence with substitution, deletion, addition, or insertion of one to three amino acids in the amino acid sequence represented by any one of SEQ ID NOs: 2 to 5.

Item 19. An oligopeptide for use as a Wnt signaling pathway inhibitor, the oligopeptide consisting of an amino acid sequence containing a DE loop sequence of RANKL protein and a β-strand D sequence of RANKL protein placed adjacent to the N-terminal side of the DE loop sequence,
wherein the DE loop sequence is the following amino acid sequence (a) or (b) :
   (a) the amino acid sequence represented by SEQ ID NO: 1, or
   (b) an amino acid sequence with substitution, deletion, addition, or insertion of one amino acid in the amino acid sequence represented by SEQ ID NO: 1; and
the β-strand D sequence is the following amino acid sequence (c) or (d) :
   (c) the amino acid sequence represented by any one of SEQ ID NOs: 2 to 5, or
   (d) an amino acid sequence with substitution, deletion, addition, or insertion of one to three amino acids in the amino acid sequence represented by any one of SEQ ID NOs: 2 to 5.

Item 20. An oligopeptide for use as a preventive or therapeutic agent for a fibrotic disease or cancer, the oligopeptide consisting of an amino acid sequence containing a DE loop sequence of RANKL protein and a β-strand D sequence of RANKL protein placed adjacent to the N-terminal side of the DE loop sequence,
wherein the DE loop sequence is the following amino acid sequence (a) or (b) :
   (a) the amino acid sequence represented by SEQ ID NO: 1, or
   (b) an amino acid sequence with substitution, deletion, addition, or insertion of one amino acid in the amino acid sequence represented by SEQ ID NO: 1; and
the β-strand D sequence is the following amino acid sequence (c) or (d) :
   (c) the amino acid sequence represented by any one of SEQ ID NOs: 2 to 5, or
   (d) an amino acid sequence with substitution, deletion, addition, or insertion of one to three amino acids in the amino acid sequence represented by any one of SEQ ID NOs: 2 to 5.

Item 21. Use of an oligopeptide for producing a Wnt signaling pathway inhibitor, the oligopeptide consisting of an amino acid sequence containing a DE loop sequence of RANKL protein and a β-strand D sequence of RANKL protein placed adjacent to the N-terminal side of the DE loop sequence,
wherein the DE loop sequence is the following amino acid sequence (a) or (b) :
   (a) the amino acid sequence represented by SEQ ID NO: 1, or
   (b) an amino acid sequence with substitution, deletion, addition, or insertion of one amino acid in the amino acid sequence represented by SEQ ID NO: 1; and
the β-strand D sequence is the following amino acid sequence (c) or (d) :
   (c) the amino acid sequence represented by any one of SEQ ID NOs: 2 to 5, or
   (d) an amino acid sequence with substitution, deletion, addition, or insertion of one to three amino acids in the amino acid sequence represented by any one of SEQ ID NOs: 2 to 5.

Item 22. Use of an oligopeptide for producing a preventive or therapeutic agent for a fibrotic disease or cancer, the oligopeptide consisting of an amino acid sequence containing a DE loop sequence of RANKL protein and a β-strand D sequence of RANKL protein placed adjacent to the N-terminal side of the DE loop sequence,
wherein the DE loop sequence is the following amino acid sequence (a) or (b) :
   (a) the amino acid sequence represented by SEQ ID NO: 1, or
   (b) an amino acid sequence with substitution, deletion, addition, or insertion of one amino acid in the amino acid sequence represented by SEQ ID NO: 1; and
the β-strand D sequence is the following amino acid sequence (c) or (d):
   (c) the amino acid sequence represented by any one of SEQ ID NOs: 2 to 5, or
   (d) an amino acid sequence with substitution, deletion, addition, or insertion of one to three amino acids in the amino acid sequence represented by any one of SEQ ID NOs: 2 to 5.

### Advantageous Effects of Invention

The present invention can provide a Wnt signaling pathway inhibitor. The present invention can also provide a preventive or therapeutic agent for fibrotic diseases or cancer.

### Brief Description of Drawings

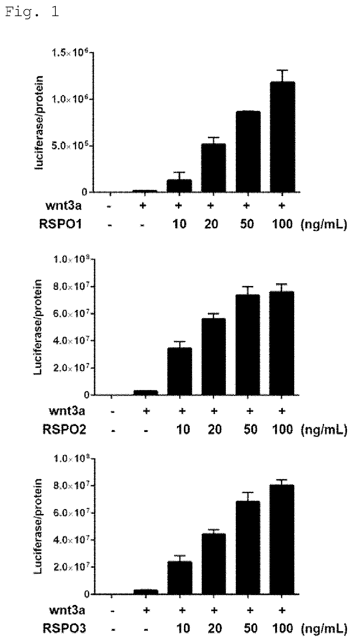

Fig. 1 shows the results without addition of RANKL peptide (MHP1-7) in Test Example 1. The vertical axis indicates luciferase activity values normalized to protein concentrations. The horizontal axis indicates the presence or absence (-/+) of the medical agents shown on the left or the concentrations thereof. Each column is the mean (n=4), and standard errors are indicated by the bars on the columns.

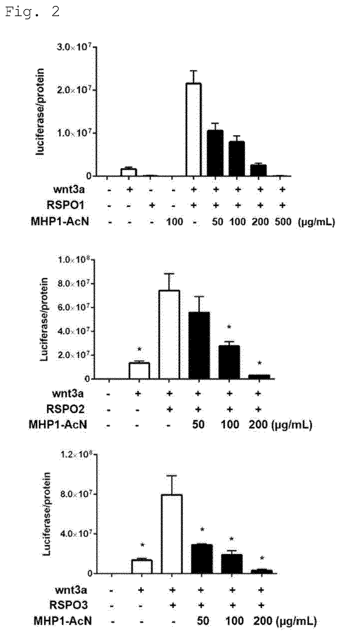

Fig. 2 shows the comparison results with and without addition of RANKL peptide (MHP1-7) in Test Example 1. The vertical axis indicates luciferase activity values normalized to protein concentrations. The horizontal axis indicates the presence or absence (-/+) of the medical agents shown on the left or the concentrations thereof. MHP1-AcN indicates MHP1-7. Each column is the mean (n=4), and standard errors are indicated by the bars on the columns. * indicates that the P value for controls (wnt3a+, RSPO1/2/3+, and MHP1-AcN (MHP1-7)-) by the ANOVA test is less than 0.05.

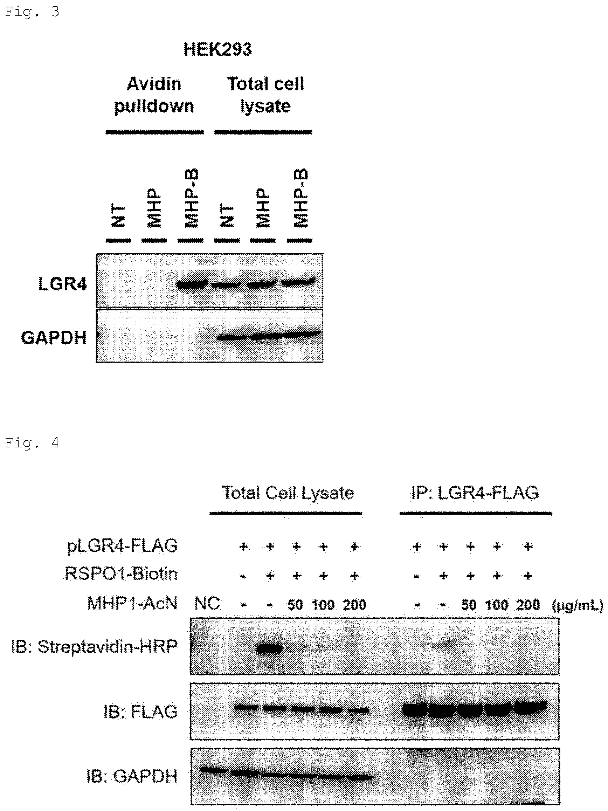

Fig. 3 shows the results of a binding test of RANKL peptide to LGR4 in Test Example 2. NC indicates no addition of RANKL peptide. MHP indicates MHP1-7, and MHP-B indicates biotinylated MHP1.
Fig. 4 shows the results of analyzing the influence of RANKL peptide on the binding of LGR4 to RSPO1 in Test Example 2. NC indicates no addition of RANKL peptide. MHP1-AcN indicates MHP1-7.

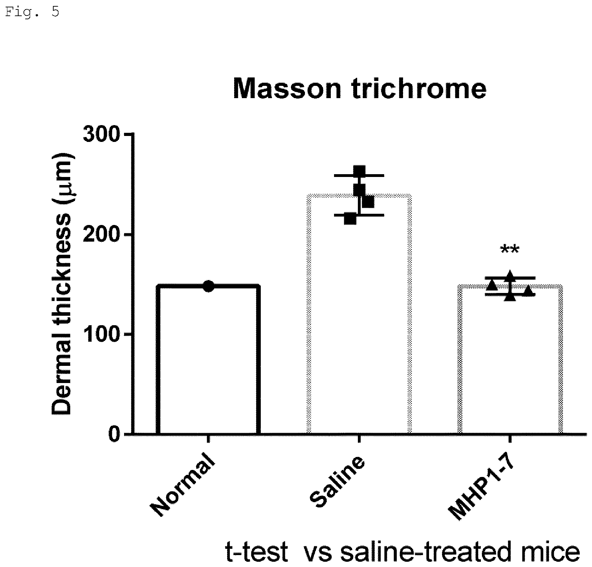

Fig. 5 shows the results of Masson's trichrome staining in Test Example 3. The vertical axis indicates the thickness of the stained area (dermis). In the horizontal axis, "Normal" indicates a group not administered with bleomycin or RANKL peptide, "Saline" indicates a group administered with bleomycin and saline, and "MHP1-7" indicates a group administered with bleomycin and MHP1-7. Each column is the mean (Normal group: n=1, others: n=4), and standard errors are indicated by the bars on the columns. ** indicates that the P value for the control (Saline) by the t-test is less than 0.01.

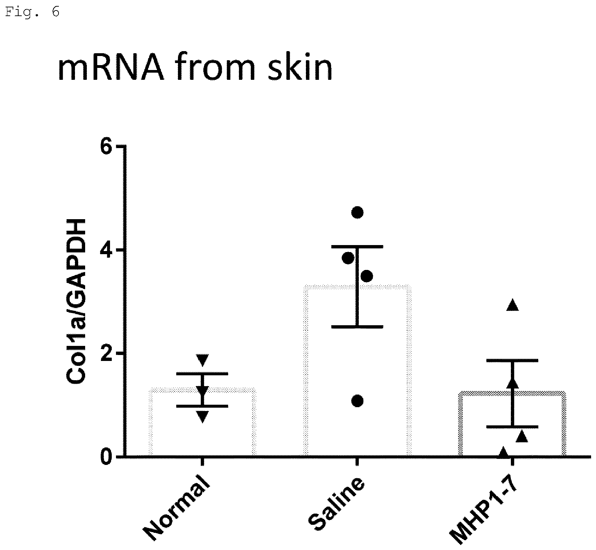

Fig. 6 shows the measurement results of collagen gene (Col1a) mRNA levels in Test Example 3. The vertical axis indicates values obtained by dividing the expression level of Colla gene by the expression level of GAPDH. In the horizontal axis, "Normal" indicates a group not administered with bleomycin or RANKL peptide, "Saline" indicates a group administered with bleomycin and saline, and "MHP1-7" indicates a group administered with bleomycin and MHP1-7. Each column is the mean (Normal group: n=1, others: n=4), and standard errors are indicated by the bars on the columns.

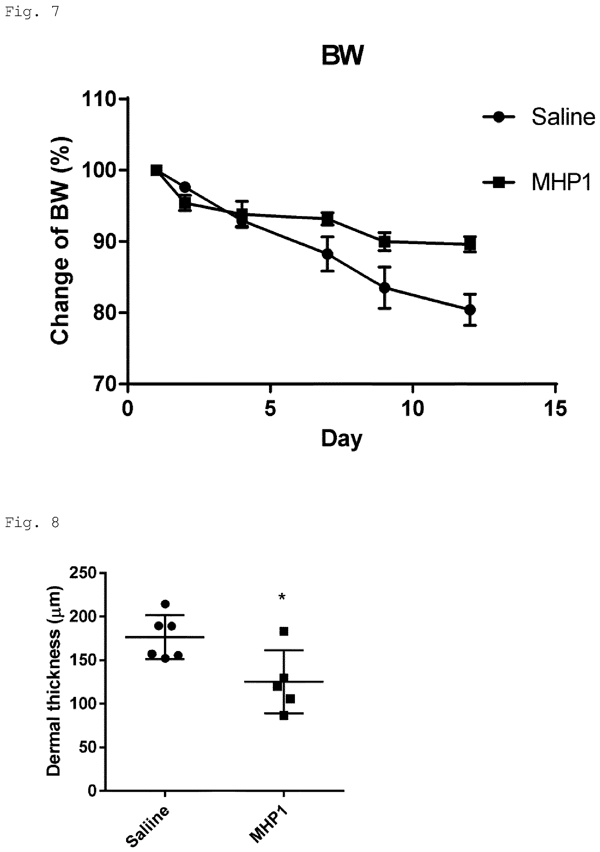

Fig. 7 shows changes in the body weight of mice in Test Example 3. The vertical axis indicates values relative to the body weight on the day of administration (Day 0), which is taken as 100%. The horizontal axis indicates the number of days elapsed from the day of administration. "Saline" indicates a group administered with bleomycin and saline (n=6), and "MHP1" indicates a group administered with bleomycin and MHP1-7 (n=6).
Fig. 8 shows the results of Masson's trichrome staining in Test Example 4. The vertical axis indicates the thickness of the stained area (dermis). In the horizontal axis, "Saline" indicates a group administered with bleomycin and saline, and "MHP1" indicates a group administered with bleomycin and MHP1-7. The middle bar is the mean (MHP1 group: n=5, Saline group: n=6), and standard error bars are shown above and below the bars. * indicates that the P value for the control (Saline) by the t-test is less than 0.05.

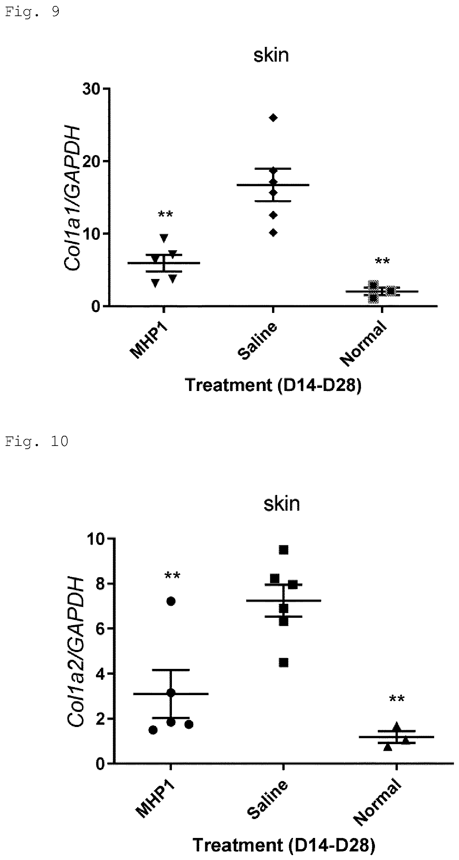

Fig. 9 shows the measurement results of collagen gene (Col1a1) mRNA levels in Test Example 4. The vertical axis indicates values obtained by dividing the expression level of Col1a1 gene by the expression level of GAPDH. In the horizontal axis, "Normal" indicates a group not administered with bleomycin or RANKL peptide, "Saline" indicates a group administered with bleomycin and saline, and "MHP1" indicates a group administered with bleomycin and MHP1-7. The middle bar is the mean (Normal group: n=3, Saline group: n=6, MHP1 group: n=5), and standard error bars are shown above and below the bars. ** indicates that the P value for the control (Saline) by the ANOVA test is less than 0.01.
Fig. 10 shows the measurement results of collagen gene (Col1a2) mRNA levels in Test Example 4. The vertical axis indicates values obtained by dividing the expression level of Col1a2 gene by the expression level of GAPDH. In the horizontal axis, "Normal" indicates a group not administered with bleomycin or RANKL peptide, "Saline" indicates a group administered with bleomycin and saline, and "MHP1" indicates a group administered with bleomycin and MHP1-7. The middle bar is the mean (Normal group: n=3, Saline group: n=6, MHP1 group: n=5), and standard error bars are shown above and below the bars. ** indicates that the P value for the control (Saline) by the ANOVA test is less than 0.01.

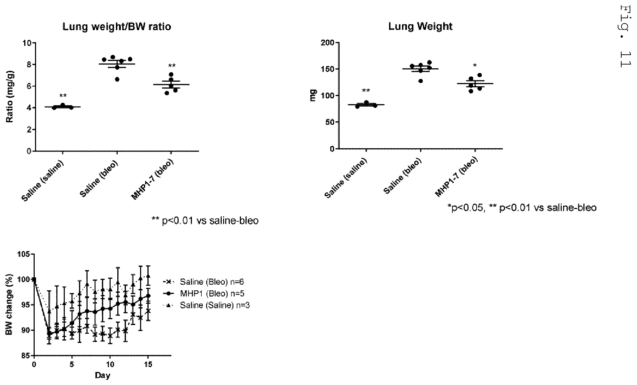

Fig. 11 shows the measurement results of lung weight and body weight in Test Example 5. The upper left graph shows the ratio of lung weight to body weight, the upper right graph shows lung weight, and the lower left graph shows changes in body weight from the day of administration. In the horizontal axis and legend, "Saline (saline)" indicates a group administered with only saline, "Saline (bleo)" indicates a group administered with bleomycin and saline, and "MHP1-7 (bleo)" (or "MHP1 (bleo)") indicates a group administered with bleomycin and MHP1-7. In the upper graph, the middle bar is the mean (Saline (saline) group: n=3, Saline (bleo) group: n=6, MHP1-7 (bleo) group: n=5), and standard error bars are shown above and below the bars. In the upper graph, * indicates that the P value for the Saline (bleo) group by the one-way ANOVA with post-hoc Dunnett's test is less than 0.05, and ** indicates that the same P value is less than 0.01. In the lower graph, the plots show the mean, and standard error bars are shown above and below the plots.

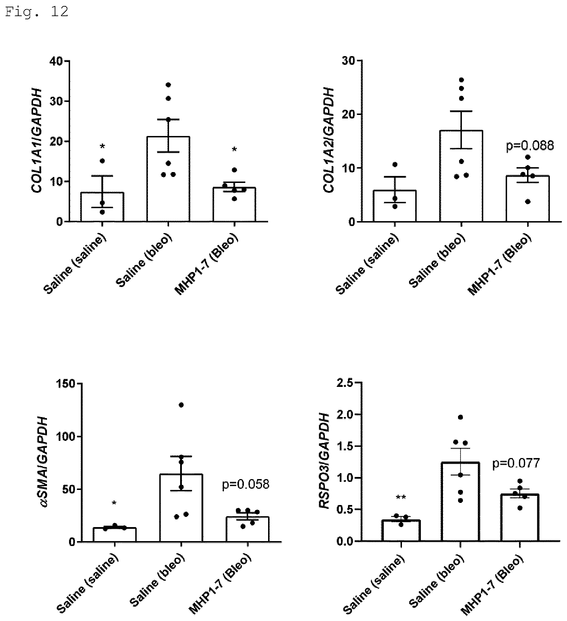

Fig. 12 shows the measurement results of fibrosis marker gene mRNA levels in Test Example 5. The vertical axis indicates values obtained by dividing the expression level of each fibrosis marker gene by the expression level of GAPDH. In the horizontal axis, "Saline (saline)" indicates a group administered with only saline, "Saline (bleo)" indicates a group administered with bleomycin and saline, and "MHP1-7 (bleo)" (or "MHP1 (bleo)") indicates a group administered with bleomycin and MHP1-7. The top of each column is the mean (Saline (saline) group: n=3, Saline (bleo) group: n=6, MHP1-7 (bleo) group: n=5), and standard error bars are shown above and below the top. * indicates that the P value for the Saline (bleo) group by the one-way ANOVA with post-hoc Dunnett's test is less than 0.05.

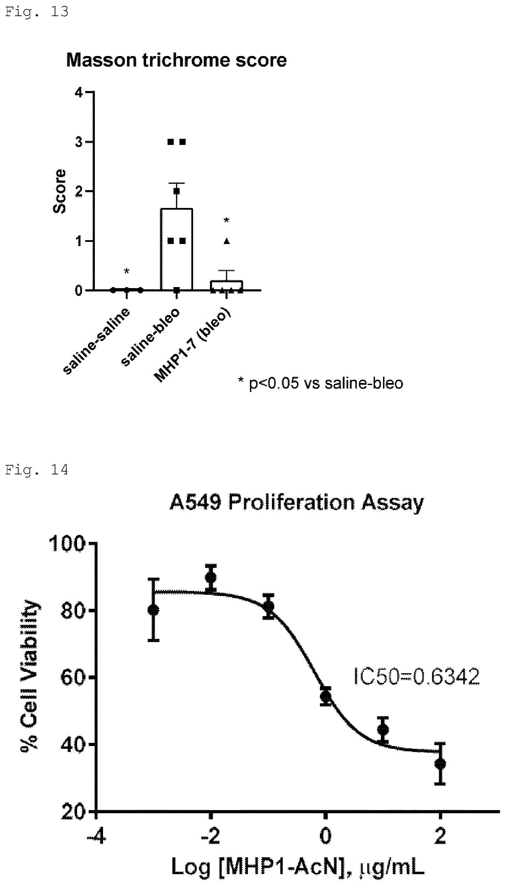

Fig. 13 shows the results of Masson's trichrome score evaluation in Test Example 5. The vertical axis indicates the Masson's trichrome score. In the horizontal axis, "Saline (saline)" indicates a group administered with only saline, "Saline (bleo)" indicates a group administered with bleomycin and saline, and "MHP1-7 (bleo)" (or "MHP1 (bleo)") indicates a group administered with bleomycin and MHP1-7. The top of each column is the mean (Saline (saline) group: n=3, Saline (bleo) group: n=6, MHP1-7 (bleo) group: n=5), and standard error bars are shown above and below the top. * indicates that the P value for the Saline (bleo) group by the one-way ANOVA with post-hoc Dunnett's test is less than 0.05.
Fig. 14 shows the results of the proliferation assay in Test Example 6-1. The vertical axis indicates the cell viability, and the horizontal axis indicates the RANKL peptide (MHP1-AcN) concentration. The plots show the mean, and standard error bars are shown above and below the plots.

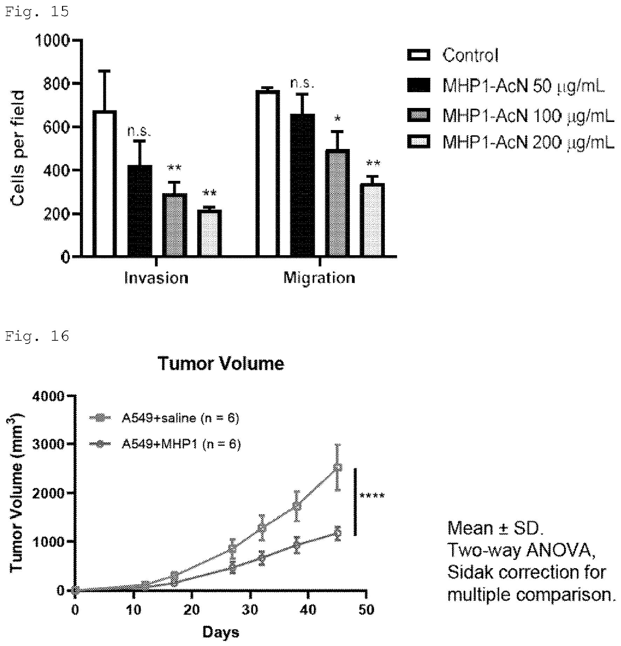

Fig. 15 shows the results of the migration/invasion assay in Test Example 6-2. The vertical axis indicates the number of cells in the observed visual field. The lower the number of cells, the more the migration/invasion is suppressed. In the legend, "Control" is a RANKL peptide (MHP1-AcN)-free group, and others are RANKL peptide (MHP1-AcN)-added groups (values are final concentrations added). The top of each column is the mean, and standard error bars are shown above and below the top. * indicates that the P value for the Control group by the one-way ANOVA with post-hoc Dunnett's test is less than 0.05, and ** indicates that the same P value is less than 0.01.
Fig. 16 shows the measurement results of the tumor volume in a cancer cell xenograft model in Test Example 6-3. The vertical axis indicates the tumor volume, and the horizontal axis indicates the number of days elapsed from the administration of cancer cells (day 0). "A549+saline" indicates a saline-administered group, and "A549+MHP1" indicates a RANKL peptide (MHP1-AcN)-administered group. The plots show the mean, and standard error bars are shown above and below the plots. **** indicates that the P-value between the two groups is less than 0.0001.

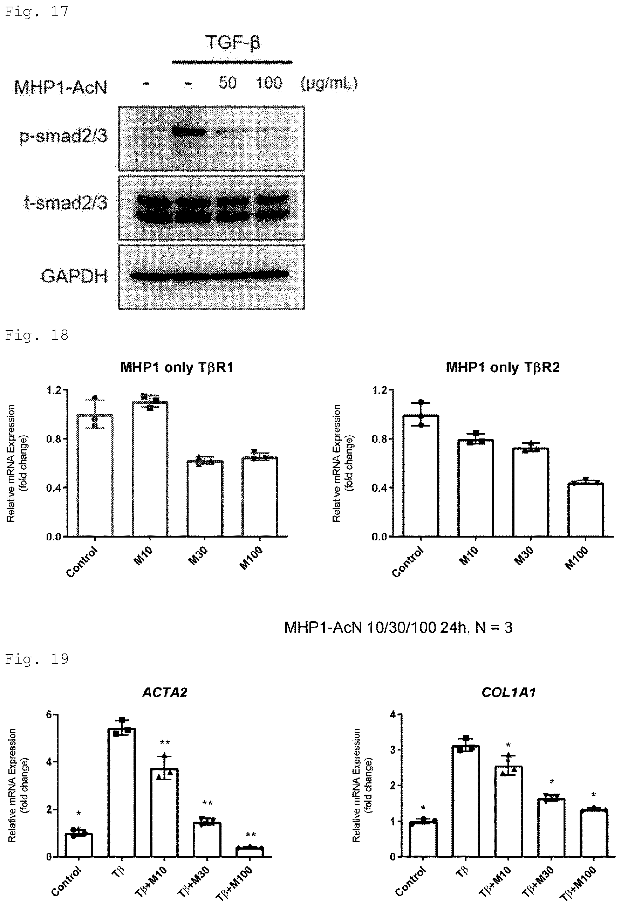

Fig. 17 shows the results of Test Example 7-1. Proteins detected are shown on the left side of the photographs. The upper part of each photograph shows the presence or absence of TGF-β administration, the presence or absence of RANKL peptide (MHP1-AcN) pretreatment, and the treatment concentration.
Fig. 18 shows the results of Test Example 7-2. The left graph shows the measurement results of TGF-β I receptor, and the right graph shows the measurement results of TGF-β II receptor. The vertical axis indicates the relative values of the expression levels. In the horizontal axis, "Control" indicates a RANKL peptide (MHP1-AcN)-free group, and "M10," "M30," and "M100" indicate RANKL peptide (MHP1-AcN)-added groups (values indicate final concentrations (unit: µg/ml)). The top of each column is the mean, and standard error bars are shown above and below the top.
Fig. 19 shows the results of Test Example 7-3. The left graph shows the measurement results of ACTA2 (α-SMA), and the right graph shows the measurement results of Col1a1. The vertical axis indicates the relative values of the expression levels. In the horizontal axis, "Control" indicates a RANKL peptide (MHP1-AcN)- and TGF-β-free group, "Tβ" indicates a TGF-β-added group, and "M10," "M30," and "M100" indicate RANKL peptide (MHP1-AcN)-added groups (values indicate final concentrations (unit: µg/ml)). The top of each column is the mean, and standard error bars are shown above and below the top. * indicates that the P value for the Tβ group by the one-way ANOVA with post-hoc Dunnett's test is less than 0.05, and ** indicates that the same P value is less than 0.01.

### Description of Embodiments

### 1. Definition

In the present specification, the amino acid sequences are all represented by single letters.

In the present specification, the terms "containing," "comprising," and "having" include the concepts of "containing," "including," "consisting essentially of," and "consisting only of."

The "identity" of amino acid sequences refers to the degree of identicalness of amino acid sequences in two or more comparable amino acid sequences. Therefore, as the identity of two amino acid sequences is higher, the identity or similarity of the sequences is higher. The level of amino acid sequence identity is determined, for example, by FASTA, which is a tool for sequence analysis, using default parameters. Alternatively, the level of amino acid sequence identity can be determined using the algorithm BLAST by Karlin and Altschul (Karlin S, Altschul SF, "Methods for assessing the statistical significance of molecular sequence features by using general scoring schemes," Proc Natl Acad Sci USA. 87: 2264-2268 (1990); and Karlin S, Altschul SF, "Applications and statistics for multiple high-scoring segments in molecular sequences," Proc Natl Acad Sci USA. 90: 5873-7 (1993)). A program called "BLASTX," which is based on this BLAST algorithm, has been developed. Specific procedures of these analysis methods are known, and reference may be made to the website (http://www.ncbi.nlm.nih.gov/) of the National Center of Biotechnology Information (NCBI). The "identity" of base sequences is also defined accordingly.

In the present specification, "conservative substitution" means that an amino acid residue is substituted with another amino acid residue having a similar side chain. For example, conservative substitutions include substitutions with amino acid residues having basic side chains, such as lysine, arginine, and histidine. Other examples of conservative substitutions include substitutions with the following amino acid residues: amino acid residues with acidic side chains, such as asparagic acid and glutamic acid; amino acid residues with uncharged polar side chains, such as glycine, asparagine, glutamine, serine, threonine, tyrosine, and cysteine; amino acid residues with nonpolar side chains, such as alanine, valine, leucine, isoleucine, proline, phenylalanine, methionine, and tryptophan; amino acid residues with β-branched side chains, such as threonine, valine, and isoleucine; and amino acid residues with aromatic side chains, such as tyrosine, phenylalanine, tryptophan, and histidine.

### 2. Oligopeptide

The following describes an oligopeptide consisting of an amino acid sequence containing a DE loop sequence of RANKL protein and a β-strand D sequence of RANKL protein placed adjacent to the N-terminal side of the DE loop sequence (which may be referred to as "the oligopeptide of the present invention" in the present specification).

The DE loop sequence of RANKL protein may be the following amino acid sequence (a) or (b):
(a) the amino acid sequence represented by SEQ ID NO: 1, or
(b) an amino acid sequence with substitution, deletion, addition, or insertion of one amino acid in the amino acid sequence represented by SEQ ID NO: 1.

The substitution in the amino acid sequence (b) is preferably conservative substitution. Further, the addition in the amino acid sequence (b) is preferably addition at the N-terminus or C-terminus.

The β-strand D sequence of RANKL protein is not particularly limited, as long as it is, among the amino acid sequences of RANKL protein, an amino acid sequence that forms β-strand D. The organism species from which the β-strand D sequence is derived is not particularly limited. Examples include various mammals, such as humans, monkeys, mice, rats, dogs, cats, and rabbits. Among these, humans, monkeys, mice, rats, etc., are preferable; humans, mice, etc., are more preferable; and humans are even more preferable. β-strand D sequences of various organism species are known. For example, those of mice and rats are disclosed in NPL 1, NPL 2, etc. Even if a β-strand D sequence derived from a certain organism species is not known, the sequence can be easily determined based on known information (e.g., NPL 1 and NPL 2). Specific examples of the β-strand D sequence of RANKL protein include the amino acid sequence represented by SEQ ID NO: 2 (part of a β-strand D sequence of mouse-derived RANKL protein), the amino acid sequence represented by SEQ ID NO: 3 (a β-strand D sequence of mouse-derived RANKL protein), the amino acid sequence represented by SEQ ID NO: 4 (part of a β-strand D sequence of human-derived RANKL protein), the amino acid sequence represented by SEQ ID NO: 5 (a β-strand D sequence of human-derived RANKL protein), and the like. The β-strand D sequence may be mutated as long as the oligopeptide of the present invention can exhibit a preventive or therapeutic effect on fibrotic diseases or cancer, an inhibitory effect on Wnt signaling pathway, and the like.

In SEQ ID NOs: 2 to 5, the leucine residues at the N-terminus of SEQ ID NOs: 2 and 4, and the leucine residue that is the fourth amino acid from the N-terminus of SEQ ID NOs: 3 and 5, are important for exhibiting a preventive or therapeutic effect on fibrotic diseases or cancer, an inhibitory effect on Wnt signaling pathway, and the like. In the β-strand D sequence, it is preferable that these leucine residues be not mutated.

The β-strand D sequence of RANKL protein is preferably the following amino acid sequence (c) or (d):
(c) the amino acid sequence represented by any one of SEQ ID NOs: 2 to 5, or
(d) an amino acid sequence with substitution, deletion, addition, or insertion of one to three amino acids in the amino acid sequence represented by any one of SEQ ID NOs: 2 to 5.

In (d) above, the number of mutated (substituted, deleted, added, or inserted) amino acids is preferably 1 or 2, and more preferably 1.

Preferred mutations in (d) above include conservative substitution. The amino acid sequence (d) is preferably the following amino acid sequence (d'):
(d') an amino acid sequence in which one to three amino acids are added to the N-terminus or C-terminus (preferably C-terminus) of the amino acid sequence represented by any one of SEQ ID NOs: 2 to 5.

In the sequence (d'), the number of amino acids added is preferably 1 or 2, and more preferably 1.

The phrase "placed adjacent to the N-terminal side" indicates that the amino acid at the N-terminus of the DE loop sequence and the amino acid at the C-terminus of the above β-strand D sequence are linked by a peptide bond.

The amino acid sequence of the oligopeptide of the present invention may contain other sequences than those described above, as long as the oligopeptide of the present invention can exhibit a preventive or therapeutic effect on fibrotic diseases or cancer, an inhibitory effect on Wnt signaling pathway, and the like. The other sequences are not particularly limited, but are preferably determined from the viewpoint, for example, that the intracellular half-life is longer. The hydrophilicity of the oligopeptide and the intracellular half-life can be presumed based on various websites (e.g., ExPASy (http://web.expasy.org/protparam/)). When ExPASy is used regarding hydrophilicity, it is preferable to design a sequence so that the "grand average of hydropathicity" shows a negative value.

The other sequences particularly preferably include a β-strand E sequence of RANKL protein, from the viewpoint of a preventive or therapeutic effect on fibrotic diseases or cancer, an inhibitory effect on Wnt signaling pathway, and the like. The β-strand E sequence is preferably placed adjacent to the C-terminal side of the DE loop sequence. The phrase "adjacent to the C-terminal side" indicates that the amino acid at the C-terminus of the DE loop sequence and the amino acid at the N-terminus of the above β-strand E sequence are linked by a peptide bond.

The β-strand E sequence of RANKL protein is not particularly limited, as long as it is, among the amino acid sequences of RANKL protein, an amino acid sequence that forms β-strand E. The organism species from which the β-strand E sequence is derived is not particularly limited. Examples include various mammals, such as humans, monkeys, mice, rats, dogs, cats, and rabbits. Among these, humans, monkeys, mice, rats, etc., are preferable; humans, mice, etc., are more preferable; and humans are even more preferable. β-strand E sequences of various organism species are known. For example, those of mice and rats are disclosed in NPL 1, NPL 2, etc. Even if a β-strand E sequence derived from a certain organism species is not known, the sequence can be easily determined based on known information (e.g., NPL 1 and NPL 2). Specific examples of the β-strand E sequence of RANKL protein include the amino acid sequence represented by SEQ ID NO: 6 (a β-strand E sequence of mouse-derived RANKL protein), the amino acid sequence represented by SEQ ID NO: 7 (part of a β-strand E sequence of mouse-derived RANKL protein), the amino acid sequence represented by SEQ ID NO: 8 (a β-strand E sequence of human-derived RANKL protein), the amino acid sequence represented by SEQ ID NO: 9 (part of a β-strand E sequence of human-derived RANKL protein), and the like. The β-strand E sequence may be mutated as long as the oligopeptide of the present invention can exhibit a preventive or therapeutic effect on fibrotic diseases or cancer, an inhibitory effect on Wnt signaling pathway, and the like.

The β-strand E sequence of RANKL protein is preferably the following amino acid sequence (e) or (f):
(e) the amino acid sequence represented by any one of SEQ ID NOs: 6 to 9, or
(f) an amino acid sequence with substitution, deletion, addition, or insertion of one to three amino acids in the amino acid sequence represented by any one of SEQ ID NOs: 6 to 9.

In (f) above, the number of mutated (substituted, deleted, added, or inserted) amino acids is preferably 1 or 2, and more preferably 1.

Preferred mutations in (f) above include conservative substitution. Further, the amino acid sequence of (f) above is preferably the following amino acid sequence (f'):
(f') an amino acid sequence in which one to three amino acids are added to the N-terminus or C-terminus (preferably N-terminus) of the amino acid sequence represented by any one of SEQ ID NOs: 6 to 9.

In (f') above, the number of amino acids added is preferably 1 or 2, and more preferably 1.

The amino acid sequence of the oligopeptide of the present invention is preferably free from a CD-loop sequence of RANKL protein.

The CD loop sequence of RANKL protein is, among the amino acid sequences of RANKL protein, an amino acid sequence that forms a CD loop. CD loop sequences of various organism species are known. For example, those of mice and rats are disclosed in NPL 1, NPL 2, etc. Specific examples of the CD loop sequence of RANKL protein include the amino acid sequence represented by SEQ ID NO: 10 (a CD loop sequence of mouse-derived RANKL protein), the amino acid sequence represented by SEQ ID NO: 11 (a CD loop sequence of human-derived RANKL protein), and the like.

The length of the oligopeptide of the present invention is not particularly limited as long as it is a general length as an oligopeptide. The length is, for example, 50 amino acid residues or less, preferably 40 amino acid residues or less, more preferably 35 amino acid residues or less, still more preferably 30 amino acid residues or less, and still more preferably 25 amino acid residues or less. The length is, for example, 11 amino acid residues or more, preferably 13 amino acid residues or more, more preferably 15 amino acid residues or more, still more preferably 20 amino acid residues or more, and still more preferably 25 amino acid residues or more. The length is preferably, for example, 11 to 50 amino acid residues, more preferably 15 to 40 amino acid residues, still more preferably 20 to 35 amino acid residues, and still more preferably 25 to 30 amino acid residues.

The amino acid sequence of the oligopeptide of the present invention is preferably the following amino acid sequence (i) or (j):
(i) the amino acid sequence represented by any one of SEQ ID NOs: 12 to 21, or
(j) an amino acid sequence with substitution, deletion, addition, or insertion of one to three amino acids in the amino acid sequence represented by any one of SEQ ID NOs: 12 to 21.

The amino acid sequence of the oligopeptide of the present invention is more preferably the following amino acid sequence (i') or (j'):
(i') the amino acid sequence represented by SEQ ID NO: 12, 17, 20, or 21, or
(j') an amino acid sequence with substitution, deletion, addition, or insertion of one to three amino acids in the amino acid sequence represented by SEQ ID NO: 12, 17, 20, or 21.

In (j) and (j') above, the number of mutated (substituted, deleted, added, or inserted) amino acids is preferably 1 or 2, and more preferably 1.

The substitution in the amino acid sequences (j) and (j') is preferably conservative substitution.

The oligopeptide of the present invention may be chemically modified as long as it can exhibit a preventive or therapeutic effect on fibrotic diseases or cancer, an inhibitory effect on Wnt signaling pathway, and the like.

The C-terminus of the oligopeptide of the present invention may be a carboxyl group (-COOH), carboxylate (-COO⁻), amide (-CONH₂), or ester (-COOR).

Examples of R in the ester include C₁₋₆ alkyl groups, such as methyl, ethyl, n-propyl, isopropyl, and n-butyl; C₃₋₈ cycloalkyl groups, such as cyclopentyl and cyclohexyl; C₆₋₁₂ aryl groups, such as phenyl and α-naphthyl; phenyl-C₁₋₂ alkyl groups, such as benzyl and phenethyl; C₇₋₁₄ aralkyl groups, such as α-naphthyl-C₁₋₂ alkyl groups (e.g., α-naphthylmethyl); pivaloyloxymethyl groups; and the like.

Furthermore, the oligopeptide of the present invention also includes oligopeptides in which the amino group of the N-terminal amino acid residue is protected by a protecting group (e.g., C₁₋₆ acyl groups such as C₁₋₆ alkanoyl, including formyl and acetyl groups), etc.

The chemical modification is preferably amidation at the C-terminus, protection at the N-terminus with an acetyl group, and the like. In particular, an oligopeptide with an acetylated N-terminus is preferable for its excellent activity. In terms of stability, an oligopeptide with an amidated C-terminus is excellent. Examples of more preferred oligopeptides include oligopeptides with an amidated C-terminus and an acetylated N-terminus. Examples of particularly preferred oligopeptides include MHP1-7 obtained by amidating the C-terminus of MHP1 and acetylating the N-terminus of MHP1, MHP6-AcN obtained by amidating the C-terminus of MHP6 and acetylating the N-terminus of MHP6, MHP24-AcN obtained by amidating the C-terminus of MHP24 and acetylating the N-terminus of MHP24, and MHP24h-AcN obtained by amidating the C-terminus of MHP24h and acetylating the N-terminus of MHP24h.

A known substance for modification may be further added to the oligopeptide of the present invention, for the purpose of improving the stability, pharmacokinetics, bioavailability, etc., of drugs. Such substances for modification include, for example, polyethylene glycol chains.

The oligopeptide of the present invention includes those having various forms, such as linear oligopeptides, branched oligopeptides, and cyclic oligopeptides, with linear oligopeptides being preferable. The oligopeptide of the present invention may be crosslinked by or based on a known means as long as it can exhibit a preventive or therapeutic effect on fibrotic diseases or cancer, an inhibitory effect on Wnt signaling pathway, and the like.

The oligopeptide of the present invention may be in the form of a pharmaceutically acceptable salt with an acid or a base. The salt is not particularly limited, as long as it is a pharmaceutically acceptable salt. Acid salts and basic salts can both be employed. Examples of acid salts include inorganic acid salts, such as hydrochloride, hydrobromate, sulfate, nitrate, and phosphate; organic acid salts, such as acetate, propionate, tartrate, fumarate, maleate, malate, citrate, methanesulfonate, and p-toluenesulfonate; amino acid salts, such as aspartate and glutamate; and the like. Examples of basic salts include alkali metal salts, such as sodium salt and potassium salt; alkaline earth metal salts, such as calcium salt and magnesium salt; and the like.

The oligopeptide of the present invention may be in the form of a solvate. The solvent is not particularly limited, as long as it is pharmaceutically acceptable. Examples include water, ethanol, glycerol, acetic acid, and the like.

The oligopeptide of the present invention can be produced by a known peptide synthesis method, depending on its amino acid sequence.

### 3. Application

Since the oligopeptide of the present invention has a preventive or therapeutic effect on fibrotic diseases or cancer, an inhibitory effect on Wnt signaling pathway, an inhibitory effect on binding of LGR4 to RSPO (e.g., RSPO1, RSPO2, and RSPO3), and an inhibitory effect on epithelial-mesenchymal transition (EMT), it can be used as an active ingredient for preventive or therapeutic agents for fibrotic diseases or cancer, Wnt signaling pathway inhibitors, binding inhibitors of LGR4 and RSPO1, epithelial-mesenchymal transition inhibitors, and the like.

In the present specification, the above medical agents are also collectively referred to as "the agent of the present invention." The agent of the present invention can be used in various fields, such as medicine. The agent of the present invention may be applied to (e.g., administered to, ingested by, inoculated into) animals and humans directly or as various compositions in combination with conventional components.

In the agent of the present invention, the oligopeptides of the present invention may be used alone or in a combination of two or more.

Fibrotic diseases are diseases in which part of the biological tissue is fibrosis. Fibrotic diseases are not particularly limited, as long as they are diseases associated with tissue fibrotic conditions or tissue fibrosis. Examples of fibrotic tissues include lungs, skin, kidneys, heart, liver, bladder, gastrointestinal tract, blood vessels, and the like; and preferably skin, lungs, and the like. Examples of fibrotic diseases include scleroderma, pulmonary fibrosis, idiopathic pulmonary fibrosis, interstitial pulmonary fibrosis, renal fibrosis, renal interstitial fibrosis, liver fibrosis, peritoneal fibrosis, myocardial fibrosis, skin fibrosis, skeletal muscle fibrosis, pancreatic fibrosis, neurofibroma, vascular fibrosis, and the like. Preferred among these are scleroderma, pulmonary fibrosis, and the like.

Examples of cancer include, but are not particularly limited to, lung cancer, gastric cancer, liver cancer, esophageal cancer, pancreatic cancer, colon cancer, biliary tract cancer, kidney cancer, bladder cancer, uterine cancer, ovarian cancer, breast cancer, prostate cancer, testicular cancer, skin cancer, leukemia, bone tumor, osteosarcoma, soft tissue tumor, multiple myeloma, malignant lymphoma, pharyngeal cancer, head and neck cancer, childhood cancer, and the like. Preferred among these is lung cancer. In one embodiment of the present invention, cancer with high expression of LGR4 and RSPO (e.g., RSPO1, RSPO2, and RSPO3) is preferred.

Wnt signaling pathway inhibitors suppress or inhibit Wnt signaling. Several Wnt signaling pathways are known, but are not particularly limited. Preferred are suppressors or inhibitors of Wnt signaling pathway through β-catenin. More preferred are suppressors or inhibitors of Wnt signal activation by RSPO (R-spondin).

The content of the active ingredient in the agent of the present invention can be suitably determined taking into consideration the type of target disease, the intended therapeutic effect, the administration method, the treatment period, the patient's age, the patient's body weight, etc. For example, the content of the active ingredient in the agent of the present invention can be about 0.0001 parts by weight to 100 parts by weight based on the entire agent of the present invention.

The administration form of the agent of the present invention is not particularly limited as long as a desired effect is exhibited. The agent can be administered to mammals, including humans through an administration route, either peroral or parenteral administration, (e.g., intravenous injection, intramuscular injection, subcutaneous administration, rectal administration, transdermal administration, and local administration). The administration form is preferably parenteral administration. The dosage form and production method of the agent for peroral or parenteral administration are well known to those skilled in the art, and the agent in any dosage form can be produced in accordance with conventional methods, for example, by mixing the active ingredient with a pharmaceutically acceptable carrier and the like.

The dosage form for parenteral administration includes injectable drugs (e.g., drip-injectable drugs, intravenously injectable drugs, intramuscularly injectable drugs, subcutaneously injectable drugs, and intradermally injectable drugs), drugs for external use (e.g., ointments, cataplasms, lotions, creams, and gels), suppositories, inhalants, eye drops, eye ointments, nasal drops, ear drops, liposome drugs, and the like. For example, an injectable drug can be prepared by dissolving the oligopeptide of the present invention in injectable distilled water; and a solubilizing agent, a buffer, a pH adjuster, a tonicity agent, a soothing agent, a preservative, a stabilizer, and the like can be optionally added thereto. The agent of the present invention may in the form of a freeze-dried formulation that is prepared when needed.

The agent of the present invention may further comprise other medical agents effective in treatment or prevention of diseases. The agent of the present invention may also optionally contain components such as a sterilizer, an antiphlogistic, a cellular stimulant, vitamins, and an amino acid.

For the carriers used in formulating the agent of the present invention, an excipient, a binder, a disintegrant, a lubricant, a colorant, and a flavoring agent typically used in this technical field can be used; and a stabilizer, an emulsifier, an absorption promoter, a surfactant, a pH adjuster, an antiseptic, an antioxidant, a filler, a moisture agent, a surface activation agent, a dispersant, a buffer, a preservative, a solubilizing agent, a soothing agent, and the like can also optionally be used.

The dose of the agent of the present invention can be determined taking into consideration various factors, such as the administration route, the type of disease, the degree of symptoms, the patient's age, gender, body weight, severity of disease, pharmacological findings such as pharmacokinetics and toxicological characteristics, use or non-use of a drug delivery system, and whether the agent is administered as part of a combinational drug with other drugs. For example, the dose of the agent of the present invention can be about 1 µg/kg (body weight) to 10 g/kg (body weight) per day. The administration schedule of the agent of the present invention can also be determined in consideration of the same factors as the dose. For example, the dose per day as described above can be administered 1 to 5 times a day to a month.

### Examples

The present invention is described in detail below based on Examples; however, the present invention is not limited to these Examples.

### Production Example 1: Synthesis of Oligopeptide

Synthesis of oligopeptides consisting of the amino acid sequences shown in Table 1 was entrusted to ILS Inc. Those other than MHP24-AcN and MHP24h-AcN have no modification at the N-terminus and C-terminus, and the N-terminus is an amino group while the C-terminus is a carboxyl group. MHP24-AcN and MHP24h-AcN are oligopeptides with an acetylated N-terminus and an amidated C-terminus. Amino acid residues in these peptides are all L-forms.

Further, synthesis of an oligopeptide obtained by replacing the methionine residue of MHP1 with a D-form (MHP1-3), an oligopeptide obtained by acetylating the N-terminus of MHP1 (MHP1-6), an oligopeptide obtained by acetylating the N-terminus of MHP1 and amidating the C-terminus of MHP1 (MHP1-7 or MHP1-AcN), and an oligopeptide obtained by amidating the C-terminus of MHP1 (MHP1-8) was entrusted to ILS Inc.

Further, synthesis of an oligopeptide obtained by acetylating the N-terminus of MHP1 and adding a biositin (biotinylated lysine) residue to the C-terminus (biotinylated MHP1) was entrusted to Bachem.

HPLC and MS confirmed that oligopeptides with the desired sequences were synthesized with high purity. Hereinafter, these peptides are also generically referred to as "the RANKL peptide."

**Table 1**

| Name | Sequence | | Species origin | Number of amino adds | SEQ ID No. |
|---|---|---|---|---|---|
| MHP1 | | LMVYVVKTSIKIPSSHNLMKGGSTKNWSGN | Mouse | 30 | 12 |
| MHP2 | DYLQLMVYWKTSIKIPSSHNLMKGGSTKN | | Mouse | 30 | 13 |
| MHP4 | | LMVYVVKTSIKIPSS | Mouse | 15 | 14 |
| hMHP4 | | LMVYVTKTSIKIPSS | Human | 15 | 15 |
| MHP5 | | LMVYVVKTSIKIPSSHNLMKGG | Mouse | 22 | 16 |
| MHP6 | | LMVYVTKTSIKIPSSHTLMKGGSTKYWSGN | Human | 30 | 17 |
| MHP12 | | LMVYVVKTSIKIPSSHNLMKGGS | Mouse | 23 | 18 |
| MHP13 | | LMVYVVKTSIKIPSSHNLMKGGSTKN | Mouse | 26 | 19 |
| MHP24 -AcN | | LMVYVVKTSIKIPSSHNLMKGGST | Mouse | 24 | 20 |
| MHP24h -AcN | | LMVYVTKTSIKIPSSHTLMKGGST | Human | 24 | 21 |

### Test Example 1: Wnt Signaling Inhibition Test

The influence of RANKL peptide on Wnt signaling was analyzed. Specifically, the following procedure was performed.

HEK293 cells expressing luciferase under the control of β-catenin responsive element were plated on a 96-well plastic plate (1×10⁴ cells/well) and incubated in the presence or absence of 50 ng/mL of recombinant mouse wnt3a (R&D System, #1324-WN) and recombinant human RSPO1 (R&D System, #4645-RS), recombinant human RSPO2 (PeproTech, #120-43), or recombinant human RSPO3 (PeproTech, #120-44). When these were added at the same time as RANKL peptide, 50 ng/mL of recombinant mouse wnt3a and 20 ng/mL of RSPO1/2/3 were used. After incubation for 24 hours, luciferase activity was analyzed using a luciferase assay system (Promega) and a microplate luminometer (Centro XS3 LB960; Berthold Technologies). The protein concentration of each sample was measured using Takara Bradford Protein Assay Kit (Takara Bio Inc.). The results of luciferase activity were normalized to the protein concentration of each sample.

The results are shown in Figs. 1 and 2. It was found that RANKL peptide inhibited Wnt signaling activation by RSPO.

### Test Example 2: Binding Test

Regarding binding of RANKL peptide to LGR4, the influence of RANKL peptide on binding of LGR4 to its ligand, RSPO1, was analyzed. Specifically, the following procedure was performed.

HEK293 cells were incubated with 100 µg/mL of RANKL peptide or biotinylated RANKL peptide for 30 minutes, followed by immunoprecipitation. For competitive binding analysis, HEK293 cells were transfected with a Flag-tagged LGR4 expression plasmid (OriGene Technologies) for 48 hours, and incubated for 2 hours in the presence of 50 ng/mL of recombinant human biotinylated RSPO1 (R&D System, #BT4645) and RANKL peptide. For immunoprecipitation, the cells were washed twice with cold PBS and dissolved in a lysis buffer (50 mM Tris-HCL, pH 7.4, 150 mM NaCl, 5 mM EDTA, 0.5% NP40, 10% glycerol and protease inhibitor cocktail (Roche)). The sample was centrifuged at 16,000 g for 10 minutes. The supernatant was collected, and the protein concentration was determined. Immunoprecipitation from samples containing equal amounts of protein was performed at 4°C overnight using high-capacity streptavidin agarose (Thermo Fisher Scientific) or Anti-FLAG M2 Affinity Gel (Sigma-Aldrich), followed by washing twice with a lysis buffer, and elution with 2X Laemmli Sample Buffer at 37°C for 30 minutes before Western blotting. The blotted membrane was incubated with the primary antibody at 4°C overnight, washed with TBS containing 0.1% Tween-20, then incubated with the HRP-conjugated secondary antibody at room temperature for 1 hour, and then incubated with Chemi-Lumi One L (Nacalai Tesque). The antibodies and reagents are as follows: anti-LGR4 (Thermo Fisher Scientific, PA5-67868), anti-GAPDH (Sigma-Aldrich, MAB374), anti-FLAG (Sigma-Aldrich, F1804), anti-mouse IgG-HRP (NA931V, GE Healthcare), anti-rabbit IgG-HRP (NA934V, GE Healthcare), and HRP-Streptavidin (Sigma-Aldrich).

The results are shown in Figs. 3 and 4. In Fig. 3, anti-LGR4 antibody bands were confirmed by avidin pulldown assay, indicating that RANKL peptide binds to LGR4. In the examination with antibody Streptoavidin-HRP in Fig. 4, the presence of RANKL peptide reduced the expression of RSPO1 in the total cell lysate and the lysate immunoprecipitated with FLAG, indicating that RANKL peptide inhibits the binding of LGR4 to RSPOL.

### Test Example 3: Early Administration Test in Bleomycin-Induced Scleroderma Model

A bleomycin-induced scleroderma model was produced, and whether the symptoms were improved by the early administration of RANKL peptide was examined. Specifically, the following procedure was performed.

As test animals, C57BL6/J mice (female, 8 weeks old) were prepared. 200 µg/mouse of bleomycin was subcutaneously injected at a frequency of 5 days/week for 14 days, and in parallel with this, 800 µg/mouse of saline or RANKL peptide was intraperitoneally administered at a frequency of 5 days/week for 14 days. The body weight was measured periodically during the dosing period. The mice were sacrificed 14 days after the start of administration, and the skin tissue of the bleomycin-administered portion was stained with Masson's trichrome to evaluate fibrosis. Further, the expression of collagen gene mRNA, which is a fibrosis index, in the skin was evaluated.

The results are shown in Figs. 5, 6, and 7. It was confirmed from tissue images that RANKL peptide inhibited skin thickening and fibrosis. Further, RANKL peptide inhibited the expression of the collagen gene and suppressed weight loss. These results indicated that RANKL peptide can suppress fibrosis in the bleomycin model.

### Test Example 4: Late Administration Test in Bleomycin-Induced Scleroderma Model

A bleomycin-induced scleroderma model was produced, and whether the symptoms were improved by the late administration of RANKL peptide was examined. Specifically, the following procedure was performed.

As test animals, C57BL6/J mice (female, 8 weeks old) were prepared. 200 µg/mouse of bleomycin was subcutaneously injected at a frequency of 5 days/week for 14 days. Subsequently, 800 µg/mouse of saline or RANKL peptide was intraperitoneally administered at a frequency of 5 days/week for 14 days. The mice were sacrificed after 14 days (28 days after the start of bleomycin administration), and the skin tissue of the bleomycin-administered portion was stained with Masson's trichrome to evaluate fibrosis. Further, the expression of collagen gene mRNA, which is a fibrosis index, in the skin was evaluated.

The results are shown in Figs. 8, 9, and 10. It was confirmed from tissue images that RANKL peptide inhibited skin thickening and fibrosis. Further, RANKL peptide inhibited the expression of the collagen gene. These results indicated that RANKL peptide can repair fibrotic tissue caused by the administration of bleomycin.

### Test Example 5: Administration Test in Bleomycin-Induced Pulmonary Fibrosis Model

A bleomycin-induced pulmonary fibrosis model was produced, and whether the symptoms were improved by the administration of RANKL peptide was examined. Specifically, the following procedure was performed.

As test animals, C57BL6/J mice (female, 8 weeks old) were prepared. Saline or RANKL peptide (800 µg/mouse) was administered intraperitoneally, and immediately thereafter, saline or bleomycin (0.05 µg/mouse) was directly administered intratracheally. The mice were sacrificed 14 days after administration, the lung weight and body weight were measured, and the lung weight/body weight ratio was determined. In addition, the lung tissue was stained with Masson's trichrome, and Masson's trichrome score evaluation (0: normal, 1: fibrotic lesions predominantly at the margin of the lung, 2: marginal or global multifocal fibrotic lesions, 3: fibrotic lesions in approximately 1/3 of the area, 4: fibrotic lesions accounting for 2/3 or more of the area) was performed based on the stained images. Further, the expression of fibrosis marker gene mRNA in lung tissue was evaluated.

The results are shown in Figs. 11, 12, and 13. It was confirmed that RANKL peptide suppressed lung weight gain and weight loss. It was also confirmed that RANKL peptide inhibited lung tissue fibrosis. Further, RANKL peptide inhibited the expression of the fibrosis marker gene. These results indicated that RANKL peptide can repair fibrotic tissue caused by the administration of bleomycin.

### Test Example 6: Analysis of Influence on Cancer

The influence of RANKL peptide on cancer was analyzed.

### Test Example 6-1

The influence of RANKL peptide on the proliferation of A549 cells (human lung alveolar basal epithelial cells: high expression of both LGR4 and RSPO3 was confirmed) was examined. Specifically, the following procedure was performed. MHP1 was administered at 1 ng/ml to 100 µg/ml to A549 cells, and the cell viability was calculated by MTT assay after 48 hours.

The results are shown in Fig. 14. It was found that RANKL peptide inhibited the proliferation of lung cancer cells. It was also confirmed that along with the inhibition of the proliferation, Wnt signaling pathway was inhibited.

### Test Example 6-2

The influence of RANKL peptide on the migration and invasion of A549 cells was examined. Specifically, the following procedure was performed. A chamber of an 8-µm polycarbonate membrane filter (Neuro Probe) was installed in the upper compartment of a Boyden chamber (48-well Chamber, Neuro Probe), A549 cells were seeded in serum-free DMEM containing MHP1-AcN, and DMEM containing 10% FBS was placed in the lower compartment, followed by incubation at 37°C for 15 hours. In the invasion assay, the upper compartment was Matrigel Invasion chamber (Corning Life Science). Similarly, A549 cells were seeded in serum-free medium containing MHP1-AcN, and DMEM containing 100 FBS was placed in the lower compartment. Cells adhering to the upper surface of each upper compartment were detached, the lower surface was stained with Diff-Quick (Sysmex), and the number of cells was counted under a microscope.

The results are shown in Fig. 15. It was found that RANKL peptide inhibited the migration and invasion of lung cancer cells. It was also confirmed that along with the inhibition of the proliferation, Wnt signaling pathway was inhibited.

### Test Example 6-3

The influence of RANKL peptide on cancer in an A549 cell xenograft model was examined. Specifically, the following procedure was performed. 2×10⁶ cells were administered subcutaneously to BALB/cAJcl-nu/nu cells. After one day, 600 µg/mouse of MHP1 was administered every day, and the tumor size was measured up to 49 days. Further, the tumor weight was measured.

The results are shown in Fig. 16. It was found that RANKL peptide inhibited the growth of cancer. The influence of RANKL peptide on the degree of weight gain was not confirmed.

### Test Example 7: Analysis of Influence on Epithelial-Mesenchymal Transition (EMT) Mechanism

The influence of RANKL peptide on the EMT mechanism was analyzed.

### Test Example 7-1

The influence of RANKL peptide on smad2/3 activation induced by TGF-β was examined. Specifically, the following procedure was performed. After pretreatment of MRC-5 cells with MHP1 for 24 hours, 5 ng/ml of TGFβ was added to the medium, the medium was collected after 10 minutes, and WB was performed.

The results are shown in Fig. 17. It was found that RANKL peptide inhibited smad2/3 activation induced by TGF-β.

### Test Example 7-2

The influence of RANKL peptide on the expression of TGF-β receptor was examined. Specifically, the following procedure was performed. MHP1 was added to the medium of MRC-5 cells, the cells were collected after 24 hours, and the expression of TβR1 and TβR2 was analyzed by real-time RT-PCR.

The results are shown in Fig. 18. It was found that RANKL peptide inhibited the expression of TGF-β receptor.

### Test Example 7-3

The influence of RANKL peptide on the expression of α-SMA and Col1a1 induced by TGF-β was examined. Specifically, the following procedure was performed. After pretreatment of MHP1 with MRC-5 cells for 24 hours, 2 ng/ml of TGFβ was added, the cells were collected after 24 hours, and mRNA levels were determined by real-time RT-PCR.

The results are shown in Fig. 19. It was found that RANKL peptide inhibited the expression of α-SMA and Col1a1 induced by TGF-β. This result indicated that RANKL peptide inhibited EMT.

Sequence Listing

## Claims

1. A Wnt signaling pathway inhibitor comprising an oligopeptide consisting of an amino acid sequence containing a DE loop sequence of RANKL protein and a β-strand D sequence of RANKL protein placed adjacent to the N-terminal side of the DE loop sequence,
wherein the DE loop sequence is the following amino acid sequence (a) or (b) :
(a) the amino acid sequence represented by SEQ ID NO: 1, or
(b) an amino acid sequence with substitution, deletion, addition, or insertion of one amino acid in the amino acid sequence represented by SEQ ID NO: 1; and
the β-strand D sequence is the following amino acid sequence (c) or (d) :
(c) the amino acid sequence represented by any one of SEQ ID NOs: 2 to 5, or
(d) an amino acid sequence with substitution, deletion, addition, or insertion of one to three amino acids in the amino acid sequence represented by any one of SEQ ID NOs: 2 to 5.

2. The inhibitor according to claim 1, wherein the DE loop sequence is the amino acid sequence (a).

3. The inhibitor according to claim 2, wherein the one to three amino acids are one or two amino acids.

4. The inhibitor according to any one of claims 1 to 3, wherein in the amino acid sequence (d), the leucine residue at the N-terminus of SEQ ID NOs: 2 and 4, and the leucine residue that is the fourth amino acid residue from the N-terminus of SEQ ID NOs: 3 and 5, are not substituted or deleted.

5. The inhibitor according to any one of claims 1 to 4, wherein the amino acid sequence of the oligopeptide contains a β-strand E sequence of RANKL protein placed adjacent to the C-terminal side of the DE loop sequence.

6. The inhibitor according to claim 5, wherein the β-strand E sequence is the following amino acid sequence (e) or (f) :
(e) the amino acid sequence represented by any one of SEQ ID NOs: 6 to 9, or
(f) an amino acid sequence with substitution, deletion, addition, or insertion of one to three amino acids in the amino acid sequence represented by any one of SEQ ID NOs: 6 to 9.

7. The inhibitor according to any one of claims 1 to 6, wherein the amino acid sequence of the oligopeptide is free from a CD loop sequence of RANKL protein.

8. The inhibitor according to any one of claims 1 to 7, wherein the amino acid sequence of the oligopeptide is the following amino acid sequence (i) or (j):
(i) the amino acid sequence represented by any one of SEQ ID NOs: 12 to 21, or
(j) an amino acid sequence with substitution, deletion, addition, or insertion of one to three amino acids in the amino acid sequence represented by any one of SEQ ID NOs: 12 to 21.

9. The inhibitor according to any one of claims 1 to 7, wherein the amino acid sequence of the oligopeptide is the following amino acid sequence (i') or (j'):
(i') the amino acid sequence represented by SEQ ID NO: 12, 17, 20, or 21, or
(j') an amino acid sequence with substitution, deletion, addition, or insertion of one to three amino acids in the amino acid sequence represented by SEQ ID NO: 12, 17, 20, or 21.

10. The inhibitor according to any one of claims 1 to 9, wherein the oligopeptide has an acetylated N-terminus and an amidated C-terminus.

11. The inhibitor according to any one of claims 1 to 10, wherein the oligopeptide has a length of 50 amino acid residues or less.

12. The inhibitor according to claim 11, wherein the oligopeptide has a length of 40 amino acid residues or less.

13. The inhibitor according to any one of claims 1 to 12, for use in prevention or treatment of a fibrotic disease, or for use in prevention or treatment of cancer.

14. The inhibitor according to claim 13, wherein the fibrotic disease is a fibrotic disease in at least one member selected from the group consisting of skin and lung, and the cancer is lung cancer.

15. A preventive or therapeutic agent for a fibrotic disease, the agent comprising an oligopeptide consisting of an amino acid sequence containing a DE loop sequence of RANKL protein and a β-strand D sequence of RANKL protein placed adjacent to the N-terminal side of the DE loop sequence,
wherein the DE loop sequence is the following amino acid sequence (a) or (b) :
(a) the amino acid sequence represented by SEQ ID NO: 1, or
(b) an amino acid sequence with substitution, deletion, addition, or insertion of one amino acid in the amino acid sequence represented by SEQ ID NO: 1; and
the β-strand D sequence is the following amino acid sequence (c) or (d) :
(c) the amino acid sequence represented by any one of SEQ ID NOs: 2 to 5, or
(d) an amino acid sequence with substitution, deletion, addition, or insertion of one to three amino acids in the amino acid sequence represented by any one of SEQ ID NOs: 2 to 5.

16. A preventive or therapeutic agent for cancer, the agent comprising an oligopeptide consisting of an amino acid sequence containing a DE loop sequence of RANKL protein and a β-strand D sequence of RANKL protein placed adjacent to the N-terminal side of the DE loop sequence,
wherein the DE loop sequence is the following amino acid sequence (a) or (b) :
(a) the amino acid sequence represented by SEQ ID NO: 1, or
(b) an amino acid sequence with substitution, deletion, addition, or insertion of one amino acid in the amino acid sequence represented by SEQ ID NO: 1; and
the β-strand D sequence is the following amino acid sequence (c) or (d) :
(c) the amino acid sequence represented by any one of SEQ ID NOs: 2 to 5, or
(d) an amino acid sequence with substitution, deletion, addition, or insertion of one to three amino acids in the amino acid sequence represented by any one of SEQ ID NOs: 2 to 5.
